# EUROPÄISCHE PATENTSCHRIFT

(11) **EP 1 814 843 B1**
(45) Veröffentlichungstag und Bekanntmachung des Hinweises auf die Patenterteilung: **24.06.2015**
(21) Anmeldenummer: 05802304.5
(22) Anmeldetag: 07.11.2005
(51) Int. Cl.: C07C 219/08, A61K 9/127, A61K 47/18

(54) **(2R)-ENANTIOMER VON DOTAP-CHLORID**
(2R)-ENANTIOMER OF DOTAP-CHLORIDE
(2R)-ENANTIOMER DU CHLORURE DE DOTAP

(30) Priorität: 26.11.2004 DE 102004057303
(43) Veröffentlichungstag der Anmeldung: 08.08.2007
(73) Patentinhaber: Merck Patent GmbH, 64293 Darmstadt (DE)
(72) Erfinder: PLATSCHER, Michael, CH-8252 Schlatt (CH); HEDINGER, Alfred, CH-8240 Thayngen (CH)
(86) Internationale Anmeldenummer: PCT/EP2005/011874
(87) Internationale Veröffentlichungsnummer: WO 2006/056312

(56) Entgegenhaltungen:
- WO-A-2005/049549
- US-A- 5 264 618
- DATABASE CA [Online] CHEMICAL ABSTRACTS SERVICE, COLUMBUS, OHIO, US; PERISSI, ILARIA ET AL: "Electron Spin Resonance and Differential Scanning Calorimetry as Combined Tools for the Study of Liposomes in the Presence of Long Chain Nitroxides" XP002368978 gefunden im STN Database accession no. 137:348712 & JOURNAL OF PHYSICAL CHEMISTRY B , 106(40), 10468-10473 CODEN: JPCBFK; ISSN: 1520-6106, 2002,
- DATABASE CA [Online] CHEMICAL ABSTRACTS SERVICE, COLUMBUS, OHIO, US; PFOHL, T. ET AL: "Biological polyelectrolyte complexes in solution and confined on patterned surfaces" XP002368979 gefunden im STN Database accession no. 136:382000 & COLLOIDS AND SURFACES, A: PHYSICOCHEMICAL AND ENGINEERING ASPECTS , 198-200, 613-623 CODEN: CPEAEH; ISSN: 0927-7757, 2002,
- RADLER J O ET AL: "STRUCTURE OF DNA-CATIONIC LOPOSOME COMPLEXES: DNA INTERCALATION IN MULTILAMELLAR MEMBRANES IN DISTINCT INTERHELICAL PACKING REGIMES", SCIENCE, AMERICAN ASSOCIATION FOR THE ADVANCEMENT OF SCIENCE, WASHINGTON, DC; US, vol. 275, 7 February 1997 (1997-02-07), pages 810-814, XP002924042, ISSN: 0036-8075, DOI: 10.1126/SCIENCE.275.5301.810
- RÄDLER ET AL.: "Structure and Interfacial Aspects of Self-Assembled Cationic Lipid-DNA Gene Carrier Complexes", LANGMUIR, vol. 14, 1989, pages 4272-4283,

## Beschreibung

Die vorliegende Erfindung betrifft enantiomerenreines (2R)-DOTAP Chlorid sowie dessen Verwendung zur Herstellung von pharmazeutischen Zusammensetzungen.

Mit DOTAP Chlorid sind oben und im Folgenden N,N,N-trimethyl-2,3-bis[[(9Z)-1-oxo-9-octadecenyl]oxy]-1-propanaminiumchlorid, auch als (Z,Z)-N,N,N-trimethyl-2,3-bis[(1-oxo-9-octadecenyl)oxy]-1-propanaminiumchlorid oder 1,2-Dioleoyloxy-3-tri-methylammoniumpropanchlorid bezeichnet, und dessen Hydrate gemeint. 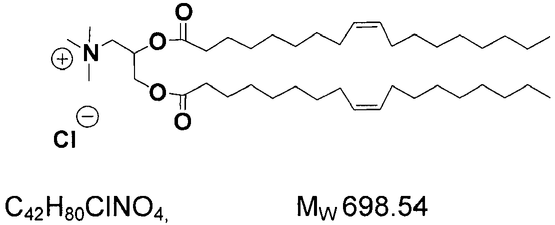 CAS Nummern: 132172-61-3 und 477274-39-8 (Racemat),
197974-73-58 (Racemat, Monohydrat)
428506-51-8 (2S-Form), 328250-28-8 (2R-Form)

Liposomen sind künstliche mehrschichtige Vesikel (kugelig in sich geschlossene Membranen) aus ambiphilen Substanzen, meist natürlichen Lipiden, in die sowohl hydrophile Substanzen in den wässrigen Innenraum verkapselt, als auch lipophile Substanzen in den Innenbereich der Lipidmembran inkorporiert werden können.

Sie werden vor allem in der Kosmetik und in der Medizin, speziell in der Dermatologie eingesetzt. Dabei werden vor allem Vitamine, Coenzyme, Haut- und Sonnenschutzmittel eingebettet. Liposomen werden in der Regel topisch angewendet.

Liposomen eine spezifischere Organverteilung erzielt werden kann, als wenn die Wirkstoffe in frei gelöster Form angewendet werden.

Wenn DNA-, RNA- oder Proteine eingeschlossen werden, erhält man Lipoplexe.

Durch Zusatz von Ölen und Einsatz von Hochdruckhomogenisatoren kann man aus Liposomen die Bildung so genannter Nanopartikel (Nanoparts) erzwingen. Dabei handelt es sich um Partikel etwa gleicher Größenordnung wie Liposomen, die aber in ihrem Inneren keine Wasserphase, sondern ein Ölphase besitzen. Sie eignen sich insbesondere für die Verkapselung von lipophilen Substanzen.

Mikroemulsionen sind kolloiddisperse, einphasige Systeme aus wässrigen, lipidartigen und tensidischen Komponenten. Sie weisen eine Teilchengrösse von 1-500 nm auf und verhalten sich ähnlich wie Flüssigkeiten.

Gerade im Zusammenhang mit den normalerweise schlecht löslichen peptidischen Wirkstoffen, Nukleotiden, Vakzine und anderer Biopharmazeutika hat der lösungsvermittelnde Effekt bei den oben beschriebenen Anwendungen eine sehr grosse Bedeutung.

Ausserdem lässt sich auf diese Weise der Abbau der Wirkstoffe im Körper bremsen und ein Sustained-Release-Effekt erzielen.

DOTAP Chlorid gehört zu der Klasse der kationischen Lipide. Diese haben, im Gegensatz zu den natürlich vorkommenden Phospholipiden, keinen zwitterionischen Charakter. Liposomen aus kationischen Lipiden weisen, allein oder kombiniert mit Phospholipiden oder anderen lipidartigen Verbindungen, eine positiv geladene Oberfläche auf. Daraus ergibt sich eine hohe Affinität zu solchen Zellen, die nach Aussen eine negativ geladene Oberfläche besitzen, beispielsweise Endothelzellen.

Besonders wichtig ist jedoch die Fähigkeit von DOTAP basierten und anderen kationischen Liposomen und Lipoplexen, in Zellen eindringen zu können und somit die in sie eingeschlossenen Wirkstoffe in das Zellinnere zu transportieren (Transfektion).

All diese Eigenschaften machen DOTAP Chlorid auch sehr interessant für die Krebstherapie. Es bietet sich durch diese Eigenschaften die Möglichkeit, in, kationische DOTAP-Liposomen eingeschlossene herkömmliche Cytostatika zu applizieren.

Die Transfektionseigenschaften von DOTAP Chlorid und anderen DOTAP-Salzen wie beispielsweise dem Acetat, Bromid, Dihydrogenphosphat, Hydrogensulfat, Iodid, Mesylat, Methylsulfat, Trifluoracetat, Sulfat oder Disulfat und Triflat sind hinreichend aus der Literatur bekannt.

DOTAP Dihydrogenphosphat und DOTAP Mesylat werden in der Literatur nur als Racemat erwähnt. Alle anderen oben genannten Salze sind jeweils als Racemat und als 2S-Enantiomer genannt, vom Chlorid und vom Methylsulfat sind zusätzlich auch die 2R-Enantiomeren erwähnt.

Bei einigen In-Vitro-Studien haben andere Salze, wie etwa DOTAP Methylsulfat, bessere Transfektionsraten erzielt als DOTAP Chlorid.

In-Vivo angewendet findet jedoch im lebenden Körper ein Anionenaustausch an der Liposomenoberfläche statt, so dass die Vorteile anderer Salze hier nicht zum Tragen kommen. Deshalb sind speziell bei der medizinischen Anwendung am Menschen und insbesondere zur parenteralen Applikation DOTAP-Salze mit physiologisch unbedenklichen Anionen, wie etwa das entsprechende Chlorid oder das Acetat, vorzuziehen.

Medizinische, insbesondere parenterale Anwendungen stellen höchste Anforderungen an die Qualität und Reinheit der verwendeten Wirk- und Hilfsstoffe. So gibt es von behördlicher Seite sehr strenge Vorschriften bezüglich der Herstellung, der Reproduzierbarkeit der Herstellung sowie des Nebenproduktprofils dieser Verbindungen. Bei parenteral angewendeten Substanzen kommt noch hinzu, dass mikrobiologische Verunreinigungen durch pathogene Keime und Endotoxine strikt zu vermeiden und zu kontrollieren sind.

DOTAP Chlorid und andere DOTAP Salze sind extrem instabil und daher an sich schwierig in einer akzeptablen Reinheit herzustellen sind, so dass sie sich für die Verwendung zur Herstellung einer Arzneimittelformulierung eignen.

Wie alle Lipide, die Ölsäurereste tragen, wie beispielsweise die natürlichen Phospholipide DOPC und DOPE, sind alle DOTAP-Salze sehr oxidationsempfindlich. Die Oxidationsprodukte ungesättigter Fettsäurederivate weisen jedoch in der Regel eine hohe Toxizität auf.

Hier sind geeignete Herstell- und Reinigungsmethoden gefordert. DOTAP Acetat liegt beispielsweise als hochsiedendes Öl vor und ist deshalb technisch nur sehr schwer in hinreichender Qualität zu erhalten.

Die üblichen Methoden zur Überwindung der Instabilität, wie z. B. die Zugabe von Oxidationsschutzmitteln in Form von Ascorbinsäure oder reduziertem L-Glutathion, schränken die generelle Anwendbarkeit von DOTAP Chlorid stark ein, weil Interaktionen mit den später einzubettenden Wirkstoffen nicht auszuschliessen sind. Ein vollständiger Sauerstoffausschluss während der Herstellung, Lagerung und Verwendung ist kaum zu verwirklichen, bzw. nur mit sehr grossem Aufwand zu ermöglichen.

DOTAP Chlorid ist kommerziell einzig als Chloroform-Lösung oder als amorpher Feststoff erhältlich.

Amorphes DOTAP Chlorid ist neben seiner Oxidatidationsempfindlichkeit auch noch extrem hygroskopisch und zerläuft bei normaler Luftfeuchtigkeit innerhalb kürzester Zeit zu einem schmierigen Film. Dies erschwert die Handhabbarkeit dieser Verbindung enorm.

So wird für amorphes DOTAP Chlorid in der Regel vom Hersteller eine Lagerung unter Schutzgas bei -20°C empfohlen und nur eine Haltbarkeit von ca. 6 Monaten garantiert.

Aus der Literatur sind lediglich verschiedene Synthesenwege für die Herstellung von amorphem, racemischem DOTAP Chlorid bekannt:
Eibel und Unger, DE4013632A1, schildern die Synthese von DOTAP Chlorid aus DOTAP Bromid durch Ionentausch im Lösungsmittelsystem Chloroform/ Methanol/wässrige HCl und anschliessende Aufreinigung mittels Chromatographie. DOTAP Bromid wird zuvor in situ aus 1-Brom-2,3-dioleoyl-oxypropan gewonnen.

Leventis und Silvius, Biochim. Biophys. Acta, 1023 (1990) 124-132, berichten über die Synthese von DOTAP Chlorid aus DOTAP Iodid durch Ionentausch im zweiphasigen System Lösungsmittel/NaCl-Lösung. DOTAP Iodid wird vorgängig durch Methylierung von der entsprechenden Dimethylamino-Verbindung mittels Methyiodid gewonnen.

Nantz et al., Biochim. Biophys. Acta, 1299 (1996) 281-283, J. Med. Chem. 40 (1997) 4069-4078, beschreiben die Synthese von DOTAP Chlorid durch nichtwässrige Ionenaustauscherchromatographie. Die gewünschte Verbindung wird durch Eindampfen des Eluates erhalten.

Felgner et al., US 5,264,618, nehmen die Methylierung der entsprechenden Dimethylamino-Verbindung direkt zu DOTAP Chlorid mittels Methylchlorid vor. Sie erhalten ein gelbes Wachs angeblich durch eine Kristallisation aus Acetonitril bei -20°C. DOTAP Chlorid ist jedoch bei Raumtemperatur in Acetonitril praktisch unlöslich. Versuche diese sogenannte Kristallisation zu reproduzieren ergaben nur amorphes Material durch Verfestigung der beim Abkühlen aus der heissen Lösung ausgeölten Substanz. Dass es sich hierbei um keine Kristallisation handelt, zeigt sich auch dadurch, dass die Autoren offenbar keinen Reinigungseffekt erzielen und die Substanz chromatographisch aufreinigen müssen.

Folglich sind bisher weder Synthesewege zur Herstellung der beiden enantiomeren DOTAP Chloride noch deren charakteristischen Eigenschaften bekannt. Durch Chemical Abstracts wurden zwar für beide Enantiomere Nummern vergeben, jedoch werden in den entsprechenden Publikationen ausschliesslich Arbeiten mit dem racemischen DOTAP Chlorid beschrieben.

Insbesondere wenn die Verbindungen für die parenterale Anwendung gedacht sind, ist eine Herstellung, welche eine Behandlung mit lonentauscherharz einschließt, äusserst problematisch im Hinblick auf mögliche mikrobiologische Verunreinigung, da entsprechende Harze ein idealer Nährboden für Keime sind und auch nach deren Abtötung noch das Risiko der Kontamination durch Endotoxine bestehen bleibt.

Aufgabe der vorliegenden Erfindung ist es daher, DOTAP Chlorid Salze und Hydrate in hoher Reinheit und mit ausreichender chemischer und physikalischer Stabilität zur Verfügung zu stellen. Eine weitere Aufgabe der vorliegenden Erfindung ist die Bereitstellung dieser Salze mit hoher Lagerstabilität, so dass sie zur Herstellung pharmazeutischer Formulierungen einsetzbar sind. Weiterhin besteht ein starkes Bedürfnis nach einem reproduzierbaren, in technischem Maßstab durchführbaren Prozess zur Herstellung von stabilen Formen von DOTAP Chlorid Salzen und Hydraten.

Enantiomerenreines DOTAP Chlorid kann aus enantiomerenreinen Edukten analog der für das Racemat beschriebenen Verfahren erhalten werden, also
über (R bzw. S)-1-Chlor-2,3-dioleoyloxypropan,
über (R bzw. S)-1-LG-2,3-dioleoyloxypropan und Ionentausch (LG=Abgangsgruppe) oder über (R bzw. S)-1-Dimethylamino-2,3-dioleoyloxypropan.

Als weitere Herstellmethode ist die Racemattrennung von racemischem DOTAP Chlorid zu nennen.

Durch Versuche wurde nun überraschend gefunden, dass sich sowohl racemisches, als auch enantiomerenreines, kristallines DOTAP Chlorid mit einer hohen chemischen Reinheit und einer ausgezeichneten Stabilität in einfacher Weise erhalten lassen. Die so erhaltenen kristallinen Produkte sind bei Raumtemperatur unter Schutzgas praktisch unbeschränkt stabil. Sie sind daher als Bestandteil oder als Ausgangsmaterial zur Herstellung von Arzneimittelformen geeignet.

Durch Versuche wurde nun überraschend gefunden, dass sich sowohl racemisches, als auch enantiomerenreines, kristallines DOTAP Chlorid mit einer hohen chemischen Reinheit und einer ausgezeichneten Stabilität in einfacher Weise erhalten lassen. Die so erhaltenen kristallinen Produkte sind bei Raumtemperatur unter Schutzgas praktisch unbeschränkt stabil. Sie sind daher als Bestandteil oder als Ausgangsmaterial zur Herstellung von Arzneimittelformen geeignet.

Gegenstand der vorliegenden Erfindung ist demnach enantiomerenreines (2R)-DOTAP Chlorid.

Stabile Kristallmodifikationen können in kristalliner und teilkristalliner Form vorliegen. Sie weisen eine bisher nie erreichte Reinheit von > 98% zusammen mit einer bisher nie erreichten Stabilität von > 98% in Bezug auf den Ausgangswert nach 12 Monaten Lagerung unter Luftausschluss bei 25°C und 60% relativer Luftfeuchtigkeit auf. (siehe dazu Tabelle 1). Die DOTAP Chlorid Kristallmodifikationen haben einen Gehalt von weniger als 1 Aequivalent Wasser oder Kristalllösungsmittel auf 1 Aequivalent DOTAP Chlorid.

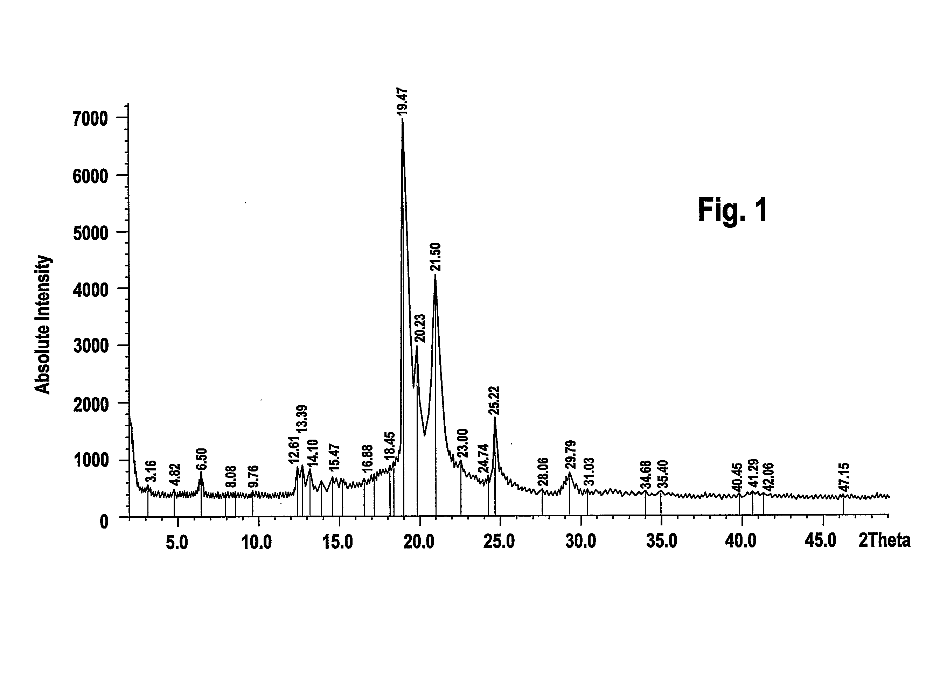

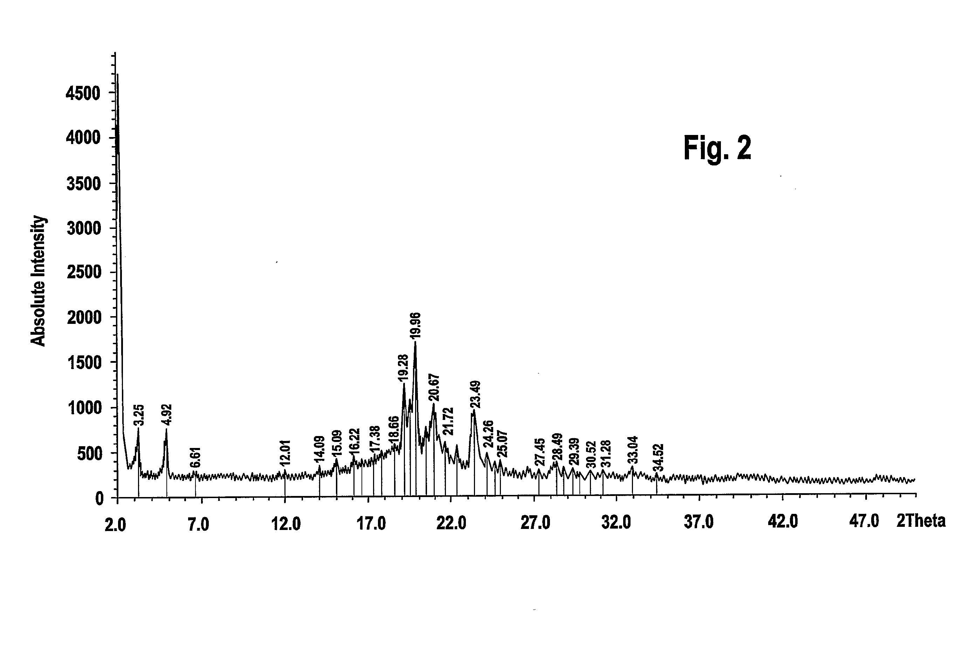

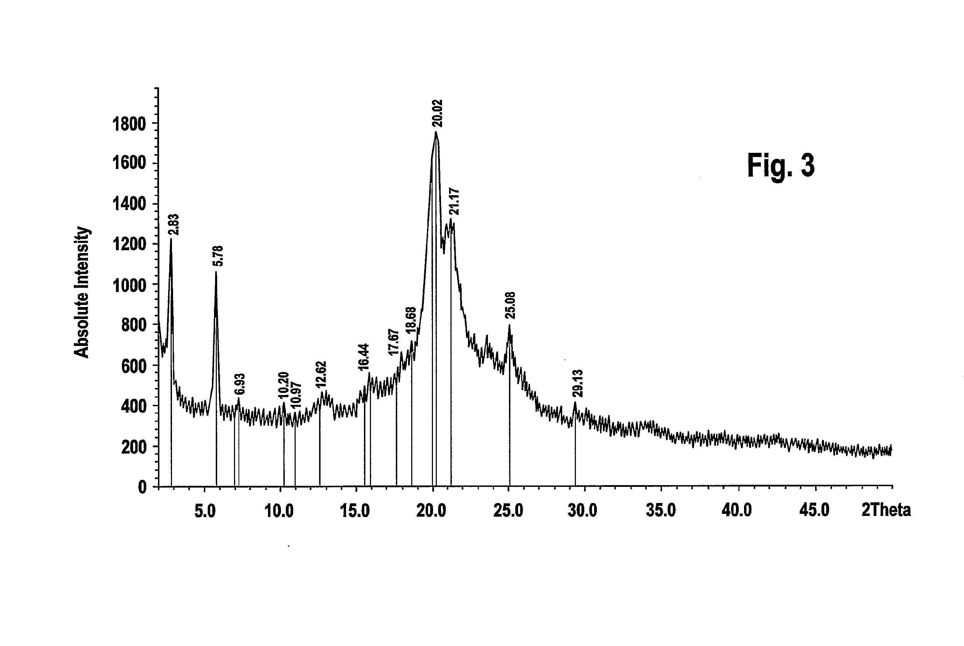

Racemische DOTAP Chlorid Kristallmodifikationen liegen beispielsweise in drei verschiedenen Kristallmodifikationen vor (Typ I, Typ II, und Typ III) und zeigen bei Pulverröntgendiffraktions-Messungen mittelscharfe Banden (siehe dazu Abb. 1 bis Abb. 3 und Tabelle 2).
Ausgewählte 2 Theta-Werte für die verschiedenen Kristallmodifikationen liegen bei 12.6, 19.5, 20.2, 21.5 und 25.2 (Typ I); bzw. 3.3, 4.9, 19.3, 20.0 und 23.5 (Typ II); bzw. 2.8, 5.8, 20.0, 21.2 und 25.1 (Typ III).

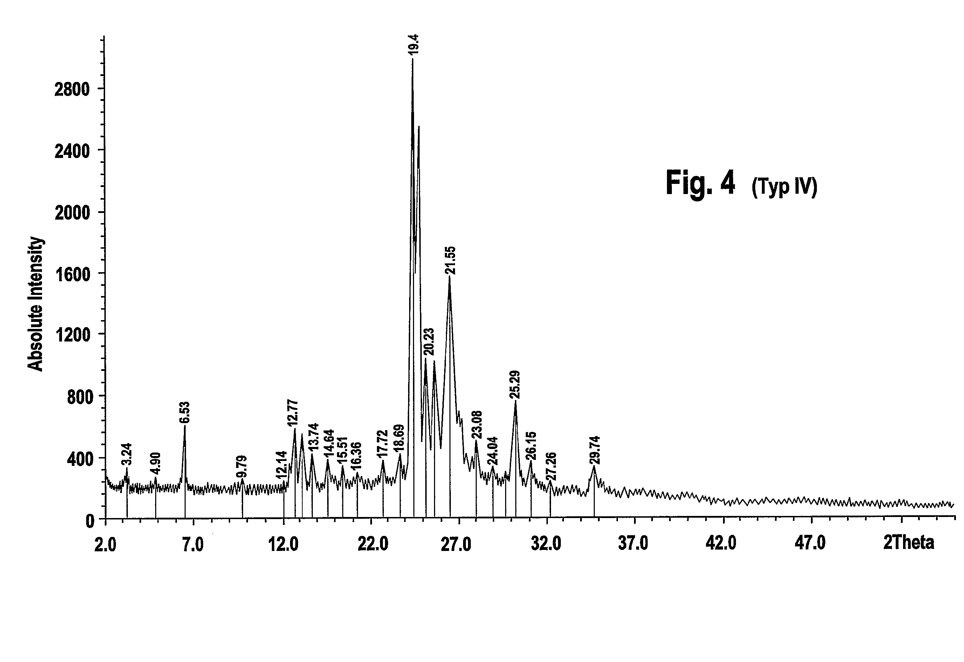

Enantiomerenreine DOTAP Chloride werden ebenfalls in kristalliner Form erhalten. Ausgewählte 2 Theta-Werte für die gefundene Kristallmodifikation liegen bei 12.8, 19.4, 19.8, 20.2, und 21.5 (Typ IV, siehe dazu Abb. 4).

Die Enantiomeren sind optisch aktiv. So besitzt (2S)-DOTAP Chlorid einen Drehwert von -2.12°, (2R)-DOTAP Chlorid von +2.12° ([α]_{D} bei 20°C, 1%ige Lösung in Dichlormethan).

Beschrieben wird im Folgenden ein Verfahren zur Herstellung von DOTAP Chlorid Kristallmodifikationen, welches dadurch gekennzeichnet ist, dass man DOTAP Chlorid aus einem aprotischen Medium auskristallisiert. Als aprotisches Medium können zu diesem Zweck aprotische Lösungsmittel oder deren Mischungen verwendet werden.

Das aprotische Medium kann auch in geringem Umfang protische Lösungsmittel, wie z. B. Wasser, enthalten. In Ausnahmefällen können unter geeigneten Bedingungen auch 25 Gew.-% protische Lösungsmittel enthalten sein. Die Kristallisation der DOTAP Chloride kann hierbei ohne vorgängige Aufreinigung direkt aus der Reaktionslösung erfolgen. Ebenso kann kristallines DOTAP Chlorid durch Umkristallisation von amorphem, teilkristallinem oder kristallinem Material gewonnen werden.

Als aprotische Lösungsmittel eignen sich vor allem
- Ether, wie z. B.: Tetrahydrofuran, Methyltetrahydrofuran, Dioxan, Diethylether, Dipropylether, Diisopropylether und Methyl-tert.-butylether
- Ketone, wie z. B.: Aceton und 2-Butanon, Methylisobutylketon, Methylisopropylketon,
- Nitrile, wie z. B.: Acetonitril und
- Ester, wie z. B.: Ethylformiat, Methylacetat, Ethylacetat, Propylacetat, Isopropylacetat, Butylacetat, Isobutylacetat, Dimethylcarbonat, Diethylcarbonat und 1,3-Dioxolidin-2-on.

Diese Lösungsmittel können jeweils rein oder im Gemisch verwendet werden, d. h. dass sowohl die verschiedenen aprotische Lösungsmittel einer Gruppe im Gemisch verwendet werden können, als auch aprotischen Lösungsmitteltypen miteinander im Gemisch eingesetzt werden können.
Wie oben schon angedeutet, können in dem verwendeten aprotischen
Lösungsmittel oder Lösungsmittelgemisch protische Lösungsmittelzusätze enthalten sein.

Solche protischen Lösungsmittelzusätze können typischerweise aus folgenden Lösungsmitteln bestehen:
- Alkohole wie z. B.: Methanol, Ethanol, n-Propanol, iso-Propanol, n- Butanol, iso-Butanol, 2-Butanol, tert-Butanol, 3-Methyl-1-butanol und Ethylenglycol, Methoxyethanol, Ethoxyethanol, oder
Wasser.
Bei den protischen Lösungsmittelzusätzen kann es sich wiederum um Zusätze der reinen Lösungsmittel oder von Gemischen dieser protischen Lösungsmittel handeln.

Die Kristallisation der DOTAP Chlorid Modifikationen wird in der Regel gezielt durch langsames Abkühlen der hergestellten Lösung auf Temperaturen unter 30°C erreicht. Die Bildung der Kristalle erfolgt entweder spontan oder durch Animpfen mit der entsprechenden DOTAP Chlorid Kristallmodifikation.

Die verschiedenen DOTAP Chlorid Kristallmodifikationen lassen sich ineinander umwandeln. Die Umwandlungen können durch Temperaturbehandlungen der isolierten Kristallmodifikationen bei erhöhter Temperatur oder durch länger andauerndes Rühren ihrer Suspensionen unter Kristallisationsbedingungen erreicht werden.

Durch den Einsatz von amorphem oder teilkristallinem DOTAP Chlorid als Ausgangsmaterial für die Umkristallisierung erhält man durch das beschriebene Verfahren im Wesentlichen kristalline DOTAP Chloride von bisher nie erreichter Reinheit zusammen mit einer bisher nie erreichten Stabilität.

Möglich ist auch die Verwendung kristalliner DOTAP Chloride zur Herstellung von Arzneimittelformulierungen, da die kristallinen DOTAP Chloride unter den gegebenen Bedingungen in fester Form eine im Zusammenwirken mit geeigneten Phospholipiden, Cholesterol und deren Derivaten neue Liposomen-Qualitäten zur Verfügung zu stellen, die im Vergleich zu herkömmlichen Formen einerseits dichtere Packungen der Lipide darstellen und andererseits einheitlicher aufgebaut sind. So zeigen Liposomen, die aus den reinen Enantiomeren hergestellt werden, im Vergleich mit den Liposomen aus racemischem DOTAP Chlorid eine um 5°C höhere Hauptphasenübergangstemperatur. Diese ist ein Mass für die Packungsdichte. Liposomen aus DOTAP Chlorid Enantiomeren weisen deshalb auch ein vermindertes Leaking der in sie eingeschlossenen Verbindung auf.

Dieses hat zur Folge, dass mit pharmazeutisch aktiven Verbindungen beladene Liposomen in der Wechselwirkung mit dem Stoffwechsel im menschlichen oder tierischen Körper Wirkstoffe verzögert abgeben werden. Vorteilhafter Weise können so insbesondere empfindliche Wirkstoffe gezielter an den gewünschten Ort, bzw. zu dem Organ transportiert werden, wo die Arzneimittelwirkung erwünscht ist.

Auch zeigen die DOTAP Chlorid Enantiomeren besonders in Verbindung mit chiralen Lipiden wie Phospholipiden, Cholesterol und deren Derivate zelllinienspezifisch unterschiedliche Transfektionseigenschaften.

Dem Fachmann ist es möglich, zur Herstellung dieser neuen Liposomen gezielt eine Form oder ein bestimmtes Mischungsverhältnis der hiermit zur Verfügung gestellten DOTAP-Chloride auszuwählen, um Liposomen mit bestimmten neuen Eigenschaften herzustellen.

Ein weiterer Gegenstand der Erfindung ist folglich auch die aus der Verwendung des beanspruchten DOTAP Chlorids resultierenden pharmazeutischen Zusammensetzungen. Solche pharmazeutischen Zusammensetzungen können (2R)-DOTAP Chlorid enthalten zusammen mit anderen pharmazeutischen Wirkstoffen und bekannten üblicherweise in der Arzneimittelzubereitung eingesetzten Hilfsstoffen sowie einem oder mehreren Lösemitteln.

Diese pharmazeutischen Zusammensetzungen können beispielsweise als Liposomen, Lipoplexe, Mikroemulsionen und Nanopartikel vorliegen und beispielsweise einen Wirkstoff aus der Gruppe der Peptide, Nukleotide, Vakzine oder Zytostatika beinhalten.

Die vorliegende Beschreibung ermöglicht es dem Fachmann die Erfindung umfassend anzuwenden. Die folgenden Beispiele dienen darüber hinaus zum besseren Verständnis und zur Veranschaulichung möglicher Varianten der Erfindung.

Alle in den folgenden Beispielen erwähnten Temperaturen sind in Grad Celsius angegeben. Wo nicht anders bezeichnet sind Gehaltsangaben als Gew.-% aufgeführt.

### Beispiele zur Illustrierung der Erfindung

### Beispiel 1

### Stabilitäten

Zur Stabilitätsbestimmung der kristallinen DOTAP Chloride werden die Substanzen zusammen mit Vergleichsmustern bei 25°C und 60% relativer Feuchte unter Luftausschluss gelagert. In periodischen Abständen wird der verbleibende Gehalt an DOTAP Chlorid gemessen und im Vergleich zum Ausgangswert angegeben.

Reinheit und Gehalt an DOTAP Chlorid werden mit HPLC bestimmt. Für Typ I werden folgende Werte gefunden:

Die Stabilitätsbestimmung kann zu einem beliebigen Zeitpunkt wiederholt werden, die in Tabelle 1 angegebenen Werte sind reproduzierbar.

**Tabelle 1:**

| (R,S)-DOTAP Chlorid kristallin Typ I | Belastungszeit in Monaten | | | | | |
|---|---|---|---|---|---|---|
| | 0 | 1 | 2 | 3 | 6 | 12 |
| Flächen% | 100% | 100.0 % | 100.0 % | 100.0 % | 100.0 % | 100.0 % |
| Gewichts% | 98.6% | 97.% | 97.9% | 97.2% | 98.2% | 98.7% |

### Beispiel 2

### [Pulverröntgendiagramme]

Zur Charakterisierung der strukturellen Eigenschaften (Kristallmodifikationen) der kristallinen DOTAP Chloride werden von diesen Substanzen Pulverröntgendiagramme (Beugungsspektren) aufgenommen. Kristalline DOTAP Chloride ergeben für Lipide relativ gut aufgelöste Spektren mit mittelscharfen Banden. Die Spektren weisen auf hohe kristalline Anteile hin. Unter dem Polarisationsmikroskop sind keine amorphen Anteile sichtbar.

Beispiele von Spektren sind in Abb. 1 (Typ I), Abb. 2 (Typ II), Abb. 3 (Typ III) und Abb. 4 (Typ IV) ersichtlich.

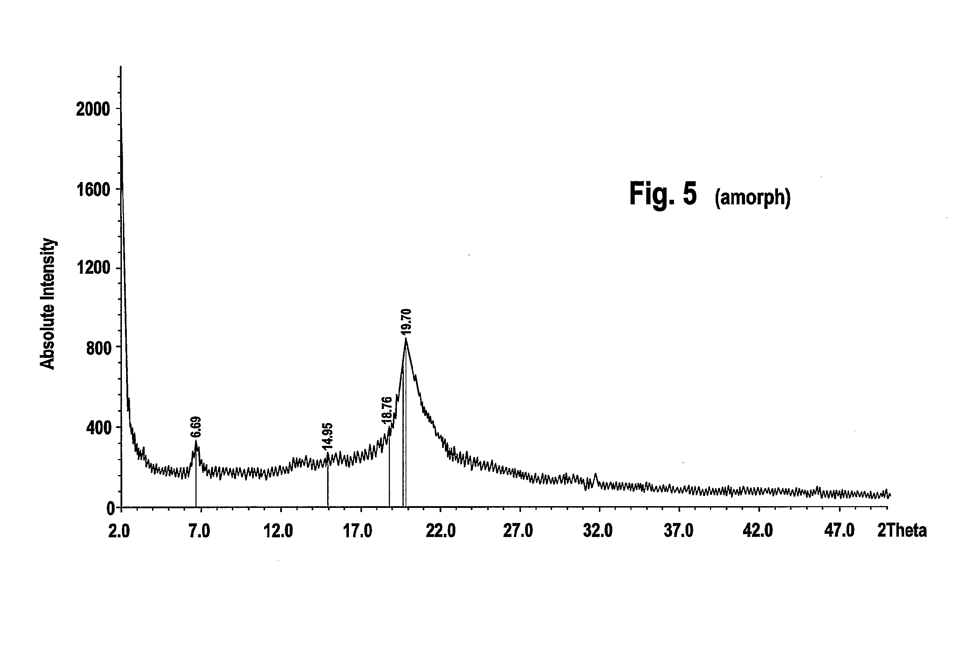

Zum Vergleich ist ein Spektrum einer kommerziell erhältlichen, amorphen Probe in Abb. 5 (amorph) aufgeführt.

In Tabelle 2 sind ausgewählte 2 Theta-Werte für die verschiedenen Kristallmodifikationen der racemischen und enantiomerenreinen DOTAP Chloride aufgelistet:

**Tabelle 2:**

| Typ | | ausgewählte 2 Theta-Werte |
|---|---|---|
| Typ I | racemisch | 12.6, 19.5, 20.2, 21.5 und 25.2 |
| Typ II | | 3.3, 4.9, 19.3, 20.0 und 23.5 |
| Typ III | | 2.8, 5.8, 20.0, 21.2 und 25.1 |
| Typ IV | enantiomerenrein | 12.8, 19.4, 19.8, 20.2, und 21.5 |

### Beispiel 3

### Hauptphasenübergangstemperaturen

Differential Scanning Caliometry (DSC) Messungen werden an multilamellaren Liposomen in Wasser vorgenommen. Von den berechneten Mengen an racemischem oder enantiomerenreinem DOTAP Chlorid werden Liposomen nach der Dünnfilmmethode hergestellt. Die Lipidkonzentration beträgt dabei jeweils 0.1 g/ml. Geeignete Mengen dieser

Dispersionen werden dann in verschliessbare Aluminiumtiegel verbracht und mit einem Kalorimeter 204 Phoenix (Netzsch, Selb, Deutschland) vermessen. Jeweils drei aufeinanderfolgende Heiz/Kühl-Verläufe von-50°C bis +20°C werden mit 1 °C/min durchgeführt.

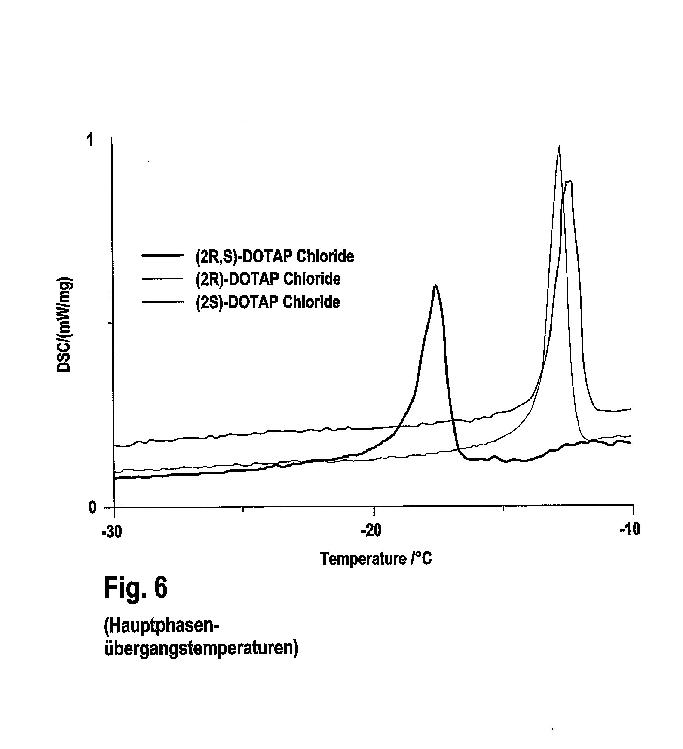

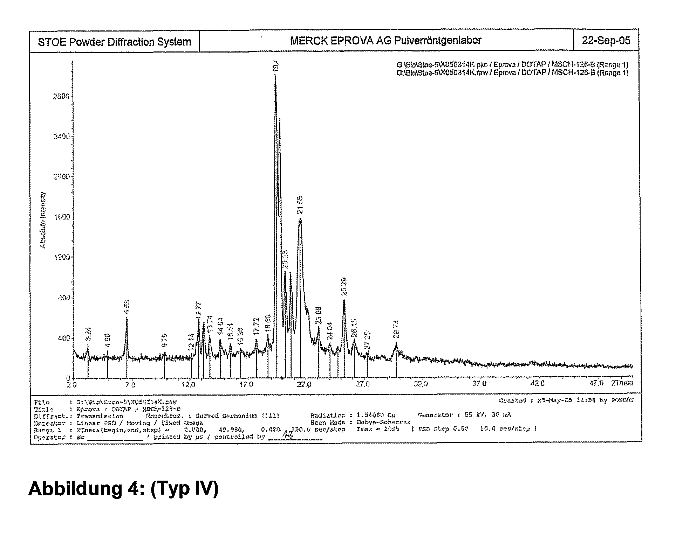

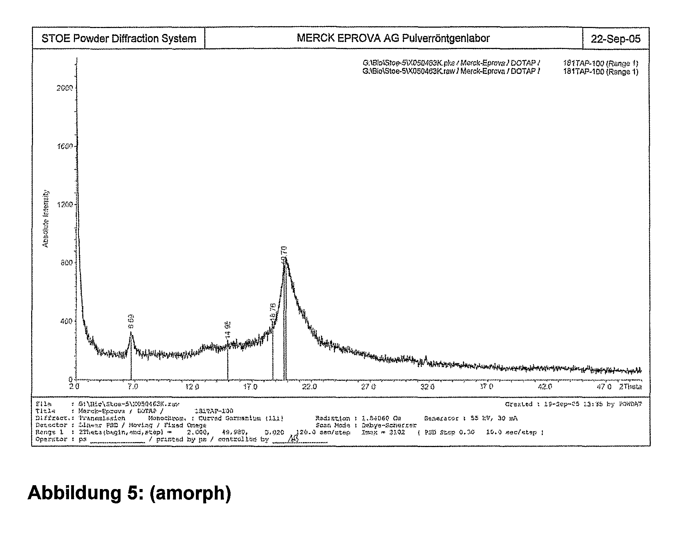

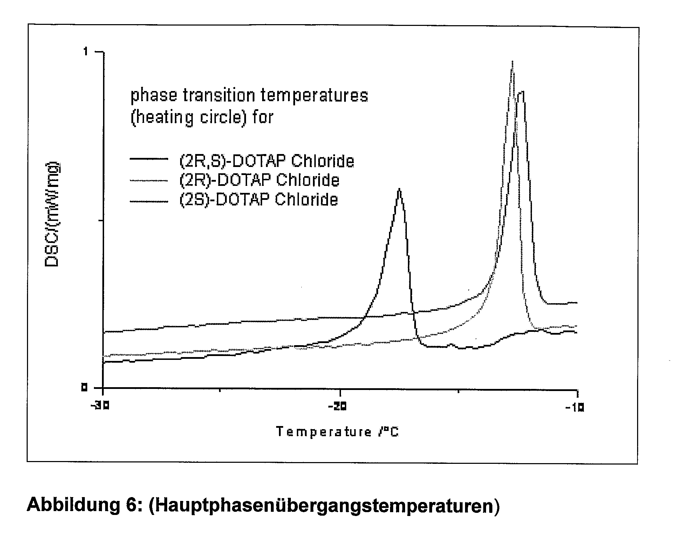

Für alle drei DOTAP Chlorid Varianten werden Phasenübergangstemperaturen unterhalb von 0°C gefunden. Für die Kühlzyklen liegt diese jeweils bei -23°C bis -24°C. Unterschiede zwischen racemischem und enantiomerenreinem DOTAP Chlorid sind jeweils bei den Heizzyklen zu sehen. (2R)-DOTAP Chlorid und (2S)-DOTAP zeigen beide einen endothermischen Phasenübergang um -12.5 °C, während für das Racemat der Phasenübergang bei -17.5 °C liegt (siehe Abb. 6).

### Beispiel 4

### Transfektionseigenschaften an COS-7-Zellen

Racemisches und enantiomerenreines DOTAP Chlorid sowie racemisches DOTAP Methylsulfat werden jeweils mit der gleichen Menge Cholesterol im Transfektionsmedium dispergiert und ultrabeschallt. Die-Liposomen und GFP-Plasmid-Lösung werden gemischt und 15 Minuten inkubiert,
Mengen pro 6well: 2 µg Plasmid/8 µg Lipid.
Nach 5 Std. Inkubation wird von den Zellen bei 37 °C/5 % CO2 der Überstand abgesaugt, 2 ml frisches Medium zugesetzt und weitere 20 Stunden inkubiert. Die FACS-Analyse ergibt nach der Aufarbeitung für alle Lipoplexe eine effiziente Transfektion. Ein signifikanter Unterschied zwischen den Transfektionsraten für die einzelnen Lipidmischungen ist festzustellen:

| | |
|---|---|
| (R)-DOTAP Chlorid/Cholesterol-Komplex: | 32.4 % |
| (S)-DOTAP Chlorid/Cholesterol-Komplex: | 11.0 % |
| (R,S)-DOTAP Chlorid/Cholesterol-Komplex: | 25.9 % |
| (R,S)-DOTAP Methylsulfat/Cholesterol-Komplex: | 20.2 % |

## Patentansprüche

1. Enantiomerenreines (2R)-1,2-Dioleoyloxy-3-trimethylammoniumpropanchlorid ((2R)-DOTAP Chlorid).

2. Verwendung von enantiomerenreinem (2R)-DOTAP Chlorid als Bestandteil zur Herstellung von Arzneimitteln.

3. Pharmazeutische Zusammensetzung enthaltend enantiomerenreines (2R)-DOTAP Chlorid zusammen mit pharmazeutischen Wirkstoffen, Hilfsstoffen oder einem Lösemittel.

4. Pharmazeutische Zusammensetzung enthaltend enantiomerenreines (2R)-DOTAP Chlorid gemäss Anspruch 3, **dadurch gekennzeichnet, dass** als pharmazeutischer Wirkstoff ein Wirkstoff, ausgewählt aus der Gruppe der Peptide, Nukleotide, Vakzine und Zytostatika, eingesetzt wird.

5. Pharmazeutische Zusammensetzung enthaltend enantiomerenreines (2R)-DOTAP Chlorid gemäss Anspruch 3, **dadurch gekennzeichnet, dass** sie Liposomen, Lipoplexe, Nanopartikel oder Mikroemulsionen aufweist.

## Claims

1. Enantiomerically pure (2R)-1,2-dioleoyloxy-3-trimethylammoniumpropane chloride ((2R)-DOTAP chloride).

2. Use of enantiomerically pure (2R)-DOTAP chloride as constituent for the preparation of medicaments.

3. Pharmaceutical composition comprising enantiomerically pure (2R)-DOTAP chloride together with pharmaceutical active compounds, adjuvants or a solvent.

4. Pharmaceutical composition comprising enantiomerically pure (2R)-DOTAP chloride according to Claim 3, **characterised in that** the pharmaceutical active compound employed is an active compound selected from the group of the peptides, nucleotides, vaccines and cytostatic agents.

5. Pharmaceutical composition comprising enantiomerically pure (2R)-DOTAP chloride according to Claim 3, **characterised in that** it comprises liposomes, lipoplexes, nanoparticles or microemulsions.

## Revendications

1. Chlorure de (2R)-1,2-dioléoyloxy-3-triméthylammoniumpropane énantiomériquement pur (chlorure de (2R)-DOTAP).

2. Utilisation de chlorure de (2R)-DOTAP énantiomériquement pur comme constituant pour la préparation de médicaments.

3. Composition pharmaceutique comprenant du chlorure de (2R)-DOTAP énantiomériquement pur conjointement avec des composés pharmaceutiques actifs, des adjuvants ou un solvant.

4. Composition pharmaceutique comprenant du chlorure de (2R)-DOTAP énantiomériquement pur selon la revendication 3, **caractérisé en ce que** le composé pharmaceutique actif employé est un composé actif choisi dans le groupe constitué par les peptides, les nucléotides, les vaccins et les agents cytostatiques.

5. Composition pharmaceutique comprenant du chlorure de (2R)-DOTAP énantiomériquement pur selon la revendication 3, **caractérisé en ce qu'**il comprend des liposomes, des lipoplexes, des nanoparticules ou des microémulsions.
